(19) Europäisches Patentamt
European Patent Office
Office européen des brevets

(11) EP 1 438 943 B1

(12) EUROPÄISCHE PATENTSCHRIFT

(45) Veröffentlichungstag und Bekanntmachung des Hinweises auf die Patenterteilung:
**12.07.2006 Patentblatt 2006/28**

(51) Int Cl.:
***A61F 13/02*** *(2006.01)* ***A61L 15/16*** *(2006.01)*

(21) Anmeldenummer: **04001126.4**

(22) Anmeldetag: **20.01.2004**

(54) **Verfahren zur Herstellung von Pflastern**

Process for the manufacture of plasters

Procédé pour la fabrication des pansements

(84) Benannte Vertragsstaaten:
**AT BE BG CH CY CZ DE DK EE ES FI FR GB GR HU IE IT LI LU MC NL PT RO SE SI SK TR**

(30) Priorität: **20.01.2003 DE 10301837**

(43) Veröffentlichungstag der Anmeldung:
**21.07.2004 Patentblatt 2004/30**

(73) Patentinhaber: **Beiersdorf AG**
**20253 Hamburg (DE)**

(72) Erfinder:
- **Trotter, Sebastian**
  **21244 Buchholz (DE)**
- **Tiedemann, Günter**
  **21769 Hollnseth (DE)**
- **Köhler, Ulrich**
  **22607 Hamburg (DE)**
- **Hartkopf, Carsten**
  **22303 Hamburg (DE)**
- **Berg, Thorsten, Dr.**
  **65116 Malang (ID)**

(56) Entgegenhaltungen:
**EP-A- 0 848 936** **EP-A- 1 121 914**

- **PATENT ABSTRACTS OF JAPAN Bd. 013, Nr. 402 (C-633), 6. September 1989 (1989-09-06) -& JP 01 144486 A (TAISHO PHARMACEUT CO LTD), 6. Juni 1989 (1989-06-06)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 438 943 B1

Printed by Jouve, 75001 PARIS (FR)

## Beschreibung

Die Erfindung umfasst ein Verfahren zur Herstellung von Wundverbänden, Pflastern, die insbesondere aus elastischen Materialien, insbesondere aus Polyurethan, bestehen. Das Verfahren ermöglicht die besonders schnelle, ökonomische und einfache Herstellung von Pflastern mit einer Wundauflage, die an ihrem gesamten Rand von einer klebenden Fixierfolie umgeben ist. Diese sogenannten "Island pads" können in beliebigen Formen verschnittoptimiert ausgestanzt werden und bestehen aus elastischem, selbsthaftendem Material.

Wundverbände bestehend aus einer Wundauflage und einem Fixierverband herzustellen ist seit vielen Jahren bekannt. Bei der großtechnischen Herstellung stehen allerdings nach wie vor Verfahren im Vordergrund, bei denen eine schmale Wundauflage aus einem beliebigen geeigneten Material zentriert auf einem deutlich breiteren, einseitig mit Klebstoff bestrichenen Fixierverband aufgebracht wird. Von diesen zweischichtigen Bändern werden Streifen in der gewünschten Breite abgeschnitten.

Ein Nachteil dieser Pflaster, die an zwei Seiten offen sind, ist, dass an diesen offenen Seiten Schmutz, Feuchtigkeit und Keime eindringen können. Dennoch sind sie auf Grund ihrer preiswerten Herstellung weiter verbreitet als Pflaster, die um ihre ganze Wundauflage herum mit einer klebenden Fixierfolie umgeben sind.

Die Herstellung von allseitig klebenden Pflastern wird bislang überwiegend dadurch erreicht, dass zunächst Wundauflagen ausgestanzt werden, die anschließend mechanisch auf einen einseitig klebenden Fixierverband platziert werden müssen. Diese Methode ist jedoch verhältnismäßig aufwendig, zeit- und kostenintensiv.

Es wurde sich daher in den letzten Jahren sehr intensiv mit der Konstruktion von Maschinen auseinandergesetzt, die darauf abzielen Pflaster vom Typ "island pad" mit hoher Geschwindigkeit und geringem Verschnitt herzustellen. Beispielsweise zeigt die EP 1 121 914 einen Lösungsansatz, der es erlaubt, rechteckige Wundauflagen auszustanzen und im selben Herstellungsprozess mit einem einseitig mit Klebstoff bestrichenen Fixierverband zu verbinden.

Derartige Herstellungsprozesse, bei denen in den verarbeiteten Materialien hohe Schub- und Zugbelastungen auftreten, sind für die heute bei der Pflasterherstellung mehr und mehr eingesetzten elastischen und/oder weichen Materialien, wie Hydrogelen, nicht geeignet.

Diese Materialien sind nicht stabil genug für eine schnelle Prozessführung und reißen daher leicht oder verformen sich im Herstellungsprozess. Ein kontinuierliches und reproduzierbares Herstellungsverfahren ist dann nicht mehr gewährleistet.

Darüber hinaus ist bislang das Problem, Wundauflagen mit runden bzw. noch aufwendigeren Formen insbesondere aus weichen Materialien wie Hydrogelen verschnittarm auszustanzen, nicht gelöst. Des weiteren gilt es die Verwendung weiterer Prozessfolien in demselben Verarbeitungsgang, um hieraus ein Pflaster des Typs island pad" herzustellen, zu vermeiden.

Eine besondere Herausforderung besteht darin, derartige Wundauflagen nicht nur aus elastischen, weichen, sondern zusätzlich aus selbsthaftenden Materialien, z. B. auf Polyurethanbasis, herzustellen und zu besagten Pflastern zu verarbeiten. Neben der Berücksichtigung der geringen Formstabilität müssen damit während des gesamten Herstellungsprozesses Vorkehrungen getroffen werden, die ein Verkleben der Wundauflage mit den Maschinenteilen verhindern, was bislang eine preiswerte Produktion dieser Produkte verhinderte.

Die Aufgabe der vorliegenden Erfindung bestand also darin, ein Herstellungsverfahren zu entwickeln, das mit hoher Prozessgeschwindigkeit ermöglicht:

a) Wundauflagen mit individueller Formgebung
b) aus elastischem, ggf. weichem Material
c) aus selbsthaftenden Materialien

in einem Verarbeitungsschritt verschnittarm auszustanzen und direkt auf den einseitig klebenden Fixierverband zu übertragen, der wiederum selbst aus einem elastischen Folienmaterial, z.B. einem Hydrogel, bestehen kann, wobei die Maschine so beschaffen sein sollte, dass auch unterschiedliche Pflasterarten ohne großen Umrüstaufwand und nach Möglichkeit auch nicht klebende und feste Materialien verarbeitet werden können.

Gelöst werden diese Aufgaben durch ein Verfahren gemäß dem Hauptanspruch. Vorteilhafte Ausführungsformen des Verfahrens sind in den Unteransprüchen dargelegt.

Es war für den Fachmann überraschend, dass ein Verfahren zur Herstellung von Pflastern, bestehend aus einer Wundauflage (6) aus selbstklebendem Material (3), einer luft- und wasserdampfdurchlässigen Barrierefolie (4) und einem Trägermaterial (9), auf dem eine Klebeschicht (8) aufgebracht ist, umfassend die Verfahrensschritte

- Führen eines bandförmigen Laminats (1), bestehend aus Wundauflagenmaterial (3) zwischen der Barrierefolie (4) und einer Prozessfolie (2), mit der Barrierefolie (4) über eine Stanzwalze (10),
- Ausstanzen beliebig konturierter Wundauflagen (6),
- Übertragen der vollständig ausgestanzten Wundauflagen (6) auf eine Vakuumwalze (11),

- Überführen der Wundauflagen (6) von der Vakuumwalze (11) mit der Barrierefolie (4) auf ein Trägermaterial (9), das mit einer Klebeschicht (8) beschichtet ist,
- wobei die Fördergeschwindigkeit des Trägermaterials (9) gegenüber der Umlaufgeschwindigkeit der Vakuumwalze (11) höher ist,
- Entfernen der Prozessfolie (2) vom Wundauflagenmaterial (3) mittels einer Vakuumwalze (12) und
- abschließend Vereinzeln der Pflaster und gegebenenfalls Eindecken und Versiegeln,

die Aufgaben vollständig löst.

Klebende hydroaktive Wundauflagen, insbesondere auf Basis von Polyurethanmatrices, benötigen zur weiteren Fixierung bzw. Randschichtverklebung Wasser undurchlässige und mit einem Schmelzhaftkleber versehene Folien oder Folienverbunde, um eine wirksame Barriere a) von der und b) zur Wunde zu gewährleisten. D.h. eine Barriere für Keime, Feuchtigkeit, Wundexudat und/oder Wirkstoffe, die durch die Wundauflage nach aussen bzw. zur Wunde wandern können. Das erfindungsgemäße Verfahren ermöglicht die Herstellung von Wundauflagen mit einer integrierten Barriereschicht, so dass der Einsatz von preiswerten offenen Fixierträgern unter weitgehender Beibehaltung der vorteilhaften elastischen Eigenschaften möglich ist.

Die bei der Herstellung dieser Pflaster sonst üblichen hohen Stanzverluste und langsamen Produktionsgeschwindigkeiten werden durch das spezielle Verfahren vermieden.

Die zur Anwendung kommenden Pflastertypen bestehen vorteilhaft aus einer geschäumten oder ungeschäumten Polyurethanmatrix, mit einem Flächengewicht von 500-1500g/$m^2$, in die zur Speicherung von Flüssigkeit ein superabsorbierendes Polymer, im Bereich von 0 - 40 Gew.%, bezogen auf die Matrix, und/oder andere Wirkstoffe eingearbeitet werden können. Auf der später zur klebenden Seite des Fixierträgers zugewandten Seite ist eine ca. 20μm starke Wasserdampf durchlässige aber Wasser undurchlässige, gegebenenfalls hochelastische, nicht klebende Polymerschicht, vorteilhaft eine Polyurethanschicht, auflaminiert. Sie bildet die Barriere zwischen der mit Wundflüssigkeit gefüllten Wundauflage und dem mit einer Klebemasse beschichteten Fixierträger.

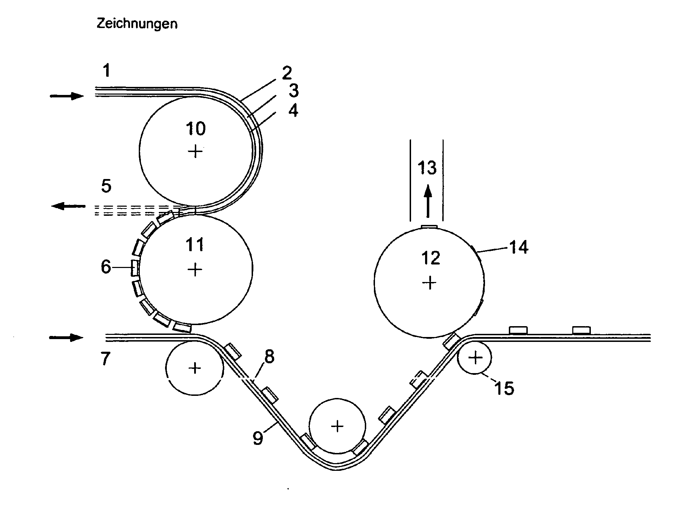

Zur besseren Erläuterung des erfindungsgemäßen Verfahrens ist in der Abbildung 1 das Verfahren zur Herstellung skizziert.

Ein wesentlicher Bestandteil der Erfindung ist die Verwendung eines geeigneten Verbandmaterials (3), das die gewünschten Eigenschaften, wie beispielsweise Elastizität und Klebrigkeit, sowie anwendungsspezifische Vorteile, wie Luft- und Wasserdampfdurchlässigkeit, besitzt. Das selbsthaftende, insbesondere auch elastische, Material (3) wird bandförmig als Laminat (1) geführt. Das Laminat (1) besteht aus dem Wundauflagenmaterial (3), das zwischen einer Barrierefolie (4) und einer Prozessfolie (2) befindlich ist. Die Barrierefolie (4) ist erfindungsgemäß wesentlich für das Verfahren und die späteren charakteristischen Eigenschaften des Pflasters. Sie muss die gewünschten Eigenschaften, wie z. B. Luft-, Wasserdampfdurchlässigkeit und gegebenenfalls Elastizität aufweisen. Die Barriereschicht (4) ist jedoch nicht selbsthaftend. Geeignete Materialien für diese Barrierefolie (4) sind z.B. Polyurethan, Copolyester-Ether, Copolyester-Ester, Polyether-Blockamid, die in vorhergehenden Arbeitsschritten auf das Wundauflagenmaterial (3) z.B. auflaminiert, gedruckt oder gesprüht werden können.

Die Prozessfolie (2) ist für die mechanische Stabilität des u.U. weichen und elastischen Verbandmaterials und der Wundauflage während des Herstellungsprozesses verantwortlich und kann z.B. aus silikonisiertem Papier, Polyester, Polypropylen gewählt werden.

Das Trägermaterial (9) besteht vorwiegend aus Textilien oder Folienmaterial aus PVC-, PE-, PET-, PU-, Polyetherester und anderen Polymeren , insbesondere aus Polyurethan. Die Wundauflage (3) selbst besteht aus Textilien, wie Vliesen oder Gewirken, Hydrogelen, Hydrocolloiden oder Polyurethangelen, die gegebenenfalls imprägniert werden können, um das Verkleben mit der Wunde zu verhindern. Die Wundauflage kann Arzneistoffe oder andere, die Wundheilung fördernde Stoffe, enthalten. Bevorzugt besteht die Wundauflage aus Polyurethan.

Die Trägerfolie (9) überlappt die Wundauflage (3) im fertigen Pflaster auf mindestens zwei gegenüberliegenden Seiten oder auf allen Seiten und ist zur Fixierung des Pflasters mit einer Klebemasse (8) versehen, die auch die Wundauflage auf dem Träger fixiert. Durch geeignete Segmentierung des überstehenden Klebebereiches kann das Pflaster insbesondere auch auf schwierig zu verklebenden Körperpartien optimal fixiert werden.

Die Stärke der Wundauflage wird den Erfordernissen des Pflasters, wie Polsterung, Saugfähigkeit und kosmetische Auffälligkeit, angepasst und beträgt bevorzugt 0,2 mm bis 10 mm und besonders bevorzugt 0,5 bis 1 mm.

Die Klebeschicht (8) kann neben Produkten auf Acrylat-Basis aus verschiedenen anderen, insbesondere unter dem Gesichtspunkt der Hautverträglichkeit geeigneten, vorzugsweise transparenten Materialien beschaffen sein. Bevorzugte Klebemassen sind Hotmelts oder lösungsmittelbasierte Klebemassen auf Basis von Naturkautschuk, Synthesekautschuk, Polyurethan und/oder Acrylat.

In einer bevorzugten Ausführungsform besteht die Trägerfolie (9) aus einer Polyurethanschicht von 0,02-0,1 mm Dicke, auf die eine hautverträgliche transparente Klebstoffschicht (8) auf Basis vernetzter Polyacrylsäurederivate von 0,02-0,08 mm Dicke aufgetragen ist.

Das, beispielsweise in der Breite der späteren Wundauflage zugeschnittene, Laminat (1) kann direkt über eine

Stanzwalze (10) geführt werden. Beim Stanzvorgang können beliebige Konturen, z.B. rund, oval, eckig, unregelmäßig geformt, zwischen der Stanzwalze (10) und der Transferwalze (11) hergestellt werden. Die Wundauflagen werden vollständig durchgestanzt und der entstehende Verschnitt wird unmittelbar von der Stanz- und Vakuumwalze weggeführt. Im einfachsten Fall, dem Ausstanzen von rechteckigen Wundauflagen, fällt kein Verschnitt an. Bei dem Einsatz rechteckiger, zentral platzierter Wundauflagen ("Island pads") wird durch den einfachen Querschneidprozess kein Stanzgitter abgezogen. Bei nicht eckigen Konturen anfallende Stanzgitter (5) werden an dieser Stelle abgeführt. Die dabei entstehenden Materialverluste durch die Randkonturen können durch den starren Verbund über die Prozessfolie (2) minimiert werden. Beispielsweise sind Stanzgitter mit nur 1-2mm Gitterbreite möglich. Die Umfangsgeschwindigkeit der Stanzwalze (10) und Vakkumwalze (11) sind bei der Ausstanzung vorteilhafterweise gegenläufig gleich groß.

In diesem Bereich ist die erfindungsgemäße vorgesehene Barrierefolie (4) vorteilhaft. Für Stanzungen ohne die Barrierefolie (4) müsste die Stanzwalze (10) mit einem handelsüblichen Releasecoating versehen werden, um den ungestörten Transfer der klebenden Matrixoberfläche (3) auf die Vakuumwalze (11) zu ermöglichen. Dieses Coating entfällt beim erfindungsgemäßen Verfahren.

Die Übertragung der ausgestanzten Wundauflagen (6) von der Stanzwalze (10) auf die Vakuumwalze (11) erfolgt vorteilhafterweise durch eine Vielzahl kleiner Luftkanäle, die senkrecht zur Oberfläche der Vakuumwalze (11) eingelassen sind. Somit werden die Stanzlinge der Wundauflage (6) auf der Oberfläche der Vakuumwalze (11) präzise festgehalten, bis sie einem geeigneten Trägermaterial (9) zugeführt und mit der nicht haftenden Oberfläche, der Barrierefolie (4), an diesem angeheftet werden. Die Oberfläche des Trägermaterials (9) ist mit einem Klebstoff (8) bestrichen, so dass die Anhaftung problemlos erfolgen kann.

Die Übertragung der ausgestanzten Wundauflagen (6) von der Vakuumwalze (11) auf das klebende Trägermaterial (7) erfolgt auf einfache Weise durch tangentialen Kontakt der klebenden Seite (8) mit der ausgestanzten Wundauflage (6) und nachfolgendem Abzug von der Vakuumwalze (11).

Der Abstand zwischen den einzelnen Wundauflagen (6) auf dem Träger (7) wird durch das Verhältnis der Transportgeschwindigkeit des Trägers (7) und der Umfangsgeschwindigkeit der Walze (11) bestimmt. Gegebenenfalls kann die exakte Positionierung der Wundauflagen (6) auf dem Träger (7) durch Anbringung einer Stützwalze in einem zur Walze (11) einstellbaren Abstand verbessert werden.

Durch die höhere Fördergeschwindigkeit des Trägers (7) gegenüber der Vakuumwalze (11) werden die ausgestanzten Wundauflagen (6) auf dem Träger (7) auseinander gezogen und es entsteht ein Pflaster des Typs "island pad".

Der Abzug der Wundauflage (6) von der Vakuumtransferwalze (11) ist durch den Kontakt des Wundauflagenmaterials (3) mit der Prozessfolie (2), nach dem Stanzen als Releaselinerabschnitt (14) gekennzeichnet, friktionsarm. Weiterhin wird die Stanzkontur der u.U. weichen, flexiblen und klebrigen Wundauflage (3) durch den Kontakt mit dem starren Releaselinerabschnitt (14) stabilisiert. Dadurch können auch komplex gestaltete Konturierungen mechanisch empfindlicher Wundauflagen spannungs- und verzerrungsfrei mit hohen Prozessgeschwindigkeiten auf einen Pflasterträger (7) laminiert werden.

Die Übergabe von der Vakuumwalze (11) auf den Fixierverband stellt insbesondere für elastische Verbandmaterialien mit filigranen Konturen einen sehr kritischen Schritt dar, da im Moment des Kontaktes der Wundauflage mit der Klebeschicht große Beschleunigungskräfte auftreten. Um diesem Umstand gerecht zu werden erfolgt die Übergabe von der Vakuumwalze auf den Fixierverband in Abhängigkeit des Wundauflagenmaterials auf folgende Weise:

Bei elastischen und konturierten Materialien (3) ist es erforderlich, den an der Vakuumwalze (11) anliegenden Unterdruck im Übergabebereich sektoriell abzuschalten bzw. auf Überdruck umzuschalten und das Trägermaterial (7) so flach an der Übergabestelle vorbeizuführen, dass die Übertragung der Wundauflage (6) auf den Träger (7) möglichst gleichzeitig auf der ganzen Fläche erfolgt.

Bei Verwendung sehr elastischen und weichen Materials und/oder Wundauflagen (3) mit sehr filigranen Konturen kann durch entsprechende Vorrichtungen die Vortriebgeschwindigkeit des Trägers (7) der Umlaufgeschwindigkeit der Vakuumwalze (11) für den Zeitpunkt der Übertragung der Wundauflage (6) von der Vakuumwalze (11) auf den Fixierverband (7) so angenähert werden, dass die Form der Wundauflage auf dem Pflaster erhalten bleibt. Entsprechende Vorrichtungen sind aus dem Stand der Technik bekannt, wie z.B. Extruder, oszillierende Fördergeschwindigkeiten etc.

Bei der Verwendung von strukturstabileren und wenig konturiert ausgestanzten Wundauflagen (3), die mit dieser Maschine ebenfalls verarbeitet werden können, wird der Träger (7) tangential mit einer gegenüber der Vakuumwalze (11) erhöhten Geschwindigkeit an dieser so dicht vorbei geführt, dass die an der Vakuumwalze (11) anhaftenden Wundauflagen (6) vom Klebebereich des Trägers (8) festgehalten und mitgenommen werden.

Die Fixierung der Wundauflage (6) auf der Klebeschicht des Trägers (8) kann durch geeignete Anpresswerkzeuge verbessert werden, bevor bei der selbsthaftenden Wundauflage im nächsten Schritt die konturiert ausgestanzte Prozessfolie (2, 14) entfernt werden muss.

Die notwendige nachträgliche Entfernung des noch auf der Wundauflage (6) verbliebenen Releaselinerabschnittes (14) kann z.B. durch Absaugung mittels einer Saugwalze (12) und Saugrohres (13) vor dem Eindecken des Pflasterverbundes mit Eindeckpapier/folie und anschließendem Vereinzeln und Siegeln auf einer konventionellen Pflastermaschine erfolgen. Gegebenenfalls kann das Abziehen der Prozessfolie (14) durch Krümmung des Verbundes von

Träger (7) und Auflage (6) mittels einer Umlenkungsrolle (15) unterhalb der Saugwalze (12) unterstützt werden.

Die Auswahl und die Entfernung der vollständig ausgestanzten Prozessfolie (2, 14) von der mit dem Träger (7) verbundenen Wundauflage (6) stellte eine weitere Herausforderung für den Fachmann dar, da folgende Bedingungen erfüllt werden müssen:

- Einerseits darf die Prozessfolie (2) nicht zu fest sein, um das auf das ggf. weiche Folienmaterial (3) abgestimmte Prägewerkzeug nicht unnötig zu belasten, gleichzeitig muss sie stabil genug sein, die große Masse der Wundauflage (3) auch bei hohen Prozessgeschwindigkeiten tragen zu können und allen im Prozess stattfindenden mechanischen Belastungen standhalten zu können.
- Ein weiteres Merkmal für die geeignete Auswahl der Prozessfolie (2) stellt deren Steifigkeit dar. Sie muss hoch genug sein, um der ausgestanzten Wundauflage (6) bis zur Fixierung auf dem Träger (7) ausreichende Stabilität zu gewähren und beim Herumführen des fertigen Pflasters über die Spendekante (15) das Abziehen von der haftenden Wundauflage zu ermöglichen.
- In diesem Zusammenhang ist auch die Klebekraft zwischen Prozessfolie (2) und Wundauflagenmaterial (3) entscheidend, die einerseits während des Herstellungsprozesses groß genug sein muss, die Wundauflage (6) auch in ausgestanzter Form zu fixieren, andererseits darf der Kraftaufwand beim Ablösen nur so groß sein, dass auch feine Konturen nicht zerstört werden.

  Die Entfernung der Prozessfolie (14) erfolgt vorteilhaft durch Vorbeiführen an einer Vakuumwalze (12) mit senkrecht zur Oberfläche verlaufenden dünnen Luftkanälen, an die ein Unterdruck angelegt ist, der das Abheben der Prozessfolienstücke (14) ermöglicht ohne gleichzeitig den Verbund aus Wundauflage (3) und Träger (7) zu zerstören. Die abgenommenen Prozessfolienstückchen (14) werden durch eine Absaugvorrichtung mit hohem Unterdruck, ggf. unterstützt durch sektorielle Abschaltung des Unterdruckes an der Vakuumwalze (12), von dieser entfernt.

Um abschließend ein gebrauchsfertiges Pflaster zu erhalten, muss der Trägerverbund (7, 3, 4) nur einer weiteren Schneidevorrichtung, bzw. zum Erhalt von runden, ovalen bzw aufwendiger geformten Pflastern einem entsprechenden Stanzwerkzeug, zugeführt werden.

Für die Abdeckung des Klebe- und Wundauflagebereiches und deren Verpackung werden dem Stand der Technik entsprechende Verfahren verwendet.

Die folgenden Vorteile ergeben sich aus dem beschriebenen erfindungsgemäßen Verfahren:

Der wichtigste Vorteil der Erfindung resultiert aus dem zweischichtigen Aufbau der selbsthaftenden Wundauflage (3, 4) die auf eine Prozessfolie (2) aufgebracht ist. Der zweischichtige Aufbau des somit nur einseitig klebenden selbstklebenden Wundauflagematerials (3) ersetzt eine sonst für die Verarbeitung von selbstklebendem Wundauflagematerial notwendige zweite Trennfolie. Das Abziehen dieser zweiten Trennfolie macht den Herstellungsprozess nach bisherigen Verfahren wesentlich aufwendiger, zeit- und kostenintensiver. Insbesondere ist auch eine exakte Positionierung der Wundauflage (6) auf dem Träger (7) nach den bekannten Verfahren nicht in der beanspruchten einfachen Art möglich.

Dementsprechend kommt der auf der selbstklebenden Seite des Wundauflagematerials (3) aufgebrachten Barrierefolie (4) eine besondere Bedeutung für den reibungslosen Ablauf des Herstellungsverfahrens zu:

1) Sie verhindert das Verkleben des selbstklebenden Wundauflagematerials (3) mit der Stanzwalze (10).
2) Sie hält die Wundauflage (6) bis zur Verklebung mit dem Träger (7) so fest, dass ein exaktes Positionieren auf dem Träger (7) möglich wird.
3) Sie schützt die sensible Wundauflage (3) bis zum Schneiden des Pflasters vor Verunreinigungen.

Der Schneidevorgang selbst kann spannungsfrei erfolgen, was für das Ausstanzen feiner Konturen sehr wichtig ist.

Die Vakuumwalze (11) ermöglicht das feste Fixieren und dadurch eine exakte Positionierung der Wundauflage (6) auf dem Träger. Dadurch wird auch das vorherige vollständige Ausstanzen der Wundauflage (6) möglich. Reststege oder unvollständiges Durchstanzen, wodurch in anderen Verfahren die Positionierung bis zur Übergabe auf den Träger gewährleistet wird, sind nicht notwendig. So lassen sich auch anspruchsvoll konturierte Wundauflagen bei minimalem Verschnitt beschädigungsfrei auf den Träger übertragen. Gleichzeitig kann durch die Fixierung der ausgestanzten Wundauflagen (6) auf der Vakuumwalze (11) der Verschnitt unmittelbar nach dem Ausstanzen vollständig entfernt werden und stört so bei späteren Arbeitsschritten nicht mehr.

Durch die Auseinzelung der Wundauflagen (6) bei der Übergabe von der Vakuumwalze (11) auf den Träger (7) kann der Verschnitt minimiert werden. Beim Schneiden rechteckiger Wundauflagen und Pflaster fällt überhaupt kein Materialabfall an.

Durch die vollständige Ablösung der Wundauflage (6) von der Vakuumwalze (11) vor der Übertragung auf den Träger (7) greift die Beschleunigung, die bei der Fixierung auf der schneller vorbeigeführten Fixierfolie hervorgerufen

wird, gleichmäßig über die ganze Wundauflage an dieser an, filigrane Konturen weicher Wundauflagen werden dadurch nicht verformt.

In Abhängigkeit des verwendeten Wundauflagematerials (3) kann durch die Verfahrensführung die Übergabe der ausgestanzten Wundauflage (6) von der Vakuumwalze (11) auf den Träger (7) vollständig spannungsfrei erfolgen, wodurch eine größt mögliche Formstabilität der ausgestanzten Wundauflage gewährleistet wird.

Durch die Verteilung der Herstellungsschritte auf unterschiedliche Walzen können diese einzeln über Motoren bewegt werden. In Kombination mit entsprechenden Kodierungen auf dem Folienmaterial, Lichtschranken und Steuereinheiten ergeben sich weitere Vorteile.

Der Transport der Folie kann praktisch spannungsfrei erfolgen, was gerade bei der Verarbeitung von weichen leicht verformbaren Materialien sehr wichtig ist.

Das Ausstanzen von Wundauflagen kann exakt mit möglicherweise aufgebrachten Motiven abgeglichen werden.

Die Positionierung der ausgestanzten Wundauflage auf dem Fixierverband kann in gleicher Weise exakt und reproduzierbar erfolgen.

Auch das Zuschneiden bzw. Ausstanzen der Pflaster kann auf diese Weise sehr exakt in Bezug auf die Wundauflage erfolgen.

Gerade für die reproduzierbare Herstellung von Pflastern bei Verwendung flexibler Materialien kommt dieser Steuerung eine große Bedeutung zu.

Die Stanzwalze (10) gibt die Größe und Form der Wundauflage (6) vor, die in Abb.1 nicht mehr dargestellte Stanzwalze zur Stanzung des fertigen Pflasters gibt die Größe des fertigen Pflasters vor. Beide Stanzwalzen können ausgewechselt werden, so dass auf einfache Weise eine Vielzahl von Kombinationsmöglichkeiten zwischen in Form und Größe variierenden Wundauflagen und Fixierverbänden mit derselben Maschine herstellbar ist.

Als Wundauflagenmaterial (3) kommen selbstklebende Materialien, die in der Wundheilung Verwendung finden, wie synthetische Polymermaterialien, zum Beispiel Polyurethane, Polyacrylate, SIBS-Massen, SEBS-Massen, Naturkautschukmassen sowie Chitosane, Alginate, Hydrogele, Hydrokolloide, insbesondere aber Polyurethane in Frage.

Erfindungsgemäß besonders hervorzuheben ist die Verwendung einer selbstklebenden Polyurethanmatrix, die als hydroaktive Wundauflage für die feuchte Wundheilung eingesetzt werden kann.

Vorzugsweise kommen elastische, vernetzte Polyurethane mit einem Masseauftragsgewicht von 50 bis 2500 $g/m^2$ zum Einsatz, wie sie zum Beispiel in der WO 97/43328 A1 beschrieben sind.

In der Regel werden Polyurethane aus den bekannten Ausgangsverbindungen der Polyurethanchemie nach bekannten Verfahren hergestellt, die in den Patenten DE-OS 3103499, DE-OS 3103500, EP 0 147 588 A1, EP 0 665 856 B1 oder DE 196 18 825 A1 dargestellt werden.

Polyurethan wird als Grundlage für das Wundauflagen- (3) und/oder Trägermaterial (9) verwendet.

Die Herstellung des Polyurethans (c) erfolgt durch die Polymerisation eines Alkohols (a)

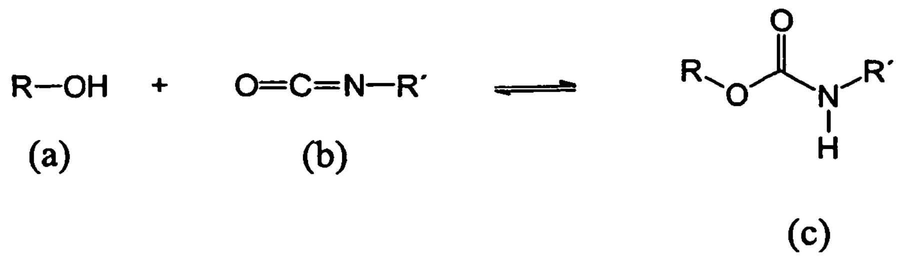

(a) (b) (c)

mit einem Isocyanat (b).

Ein entscheidender Vorteil der Polyurethanpolymer- oder gelmatrizes sind ihre selbstklebenden Eigenschaften, die ein zusätzliches Aufbringen einer Adhäsionsschicht auf die Matrix, zur Fixierung des Polymermaterials im Bereich der Haut, überflüssig machen. Im einfachsten Fall befindet sich die Polyurethan matrix zwischen einer mit ihr fest verankerten Abdeckschicht, auch als Trägerschicht benannt, und einer abziehbaren Trennschicht.

Die abziehbare Trennschicht dient zur Sicherung der Klebeschicht, zur Verbesserung der Transport- und Lagerstabilität und wird vor dem Pflastereindecken entfernt.

Geeignete Polyurethane als Matrix sind Gegenstand der DE 196 18 825, in der hydrophile, selbstklebende Polyurethangele offenbart werden, die bestehen aus

a) 2 bis 6 Hydroxylgruppen aufweisenden Polyetherpolyolen mit OH-Zahlen von 20 bis 112 und einem Ethylenoxid (EO)-Gehalt von ≥ 10 Gew.%,

b) Antioxidantien,

c) in den Polyolen a) löslichen Wismut-(III)-Carboxylaten auf Basis von Carbonsäuren mit 2 bis 18 C-Atomen als Katalysatoren sowie

d) Hexamethylendiisocyanat, mit einem Produkt der Funktionalitäten der Polyurethanbildenden Komponenten a) und d) von mindestens 5,2, wobei die Katalysatormenge c) 0,005 bis 0,25 Gew.%, bezogen auf das Polyol a) beträgt, die Menge an Antioxidantien b) im Bereich von 0,1 bis 1,0 Gew.%, bezogen auf Polyol a) liegt und ein Verhältnis von freien NCO-Gruppen der Komponente d) zu den freien OH-Gruppen der Komponente a) (Isocyanatkennzahl) im Bereich von 0,30 bis 0,70 gewählt wird.

Es werden bevorzugt 3 bis 4, ganz besonders bevorzugt 4-Hydroxylgruppen aufweisende Polyetherpolyole eingesetzt mit einer OH-Zahl im Bereich von 20 bis 112, bevorzugt 30 bis 56. Der Ethylenoxidgehalt liegt bei den erfindungsgemäß eingesetzten Polyetherpolyolen bei vorzugsweise ≥ 20 Gew.%.

Die Polyetherpolyole sind als solche an sich bekannt und werden zum Beispiel durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind zum Beispiel in High Polymers, Vol. XVI, "Polyurethanes, Chemistry and Technology" (Saunders-Frisch, Interscience Publishers, New York, Bd 1, 1962, S. 32-42) aufgeführt.

Als Isocyanatkomponente wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit 2 oder > 2 reaktionsfähigen H-Atomen und Epoxiden, wie Ethylenoxid oder Propylenoxid einer OH-Zahl von ≤ 850, bevorzugt 100 bis 600, modifiziertes Hexamethylendiisocyanat eingesetzt. Bevorzugt ist der Einsatz von modifiziertem Hexamethylendüsocyanat, insbesondere durch Prepolymerisierung mit Polyetherdiolen der OH-Zahl 200 bis 600 modifiziertes Hexamethylendiisocyanat. Ganz besonders bevorzugt sind Modifizierungen des Hexamethylendiisocyanats mit Polyetherdiolen der OH-Zahl 200-600, deren Restgehalt an monomeren Hexamethylendiisocyanat unter 0,5 Gew.% liegt.

Als Katalysatoren kommen für die erfindungsgemäßen Polyurethangele in den wasserfreien Polyetherpolyolen a) lösliche Wismut(III)-Carboxylate auf Basis linearer, verzweigter, gesättigter oder ungesättigter Carbonsäuren mit 2 bis 18, vorzugsweise 6 bis 18 C-Atomen in Frage. Bevorzugt sind Bi(III)Salze verzweigter gesättigter Carbonsäuren mit tertiären Carboxylgruppen, wie der 2,2-Dimethyl- Octansäure (zum Beispiel Versatic-Säuren, Shell). Gut geeignet sind Zubereitungen dieser Bi(III)Salze in überschüssigen Anteilen dieser Carbonsäuren. Hervorragend bewährt hat sich eine Lösung von 1 mol des Bi(III)Salzes der Versatic 10-Säure (2,2-Dimethyloctansäure) in einem Überschuß von 3 mol dieser Säure mit einem Bi-Gehalt von ca. 17%.

Es werden die Katalysatoren bevorzugt in Mengen von 0,03 bis 0,1 Gew.%, bezogen auf das Polyol a), eingesetzt.

Als Antioxidantien kommen für die erfindungsgemäßen Polyurethangele insbesondere sterisch gehinderte phenolische Stabilisatoren, wie BHT (2,6-Di-tert.butyl-4-methylphenol), Vulkanox BKF (2,2 min -Methylen-bis-(6-tert.-butyl-4-methyl phenol) (Bayer AG), Irganox 1010 (Pentaerythrityl-tetrakis-[3-(3,5-ditert.-butyl-4- hydroxyphenyl)-propionat]), Irganox 1076 (Octadecyl-3-(3,5-ditert.-butyl-4- hydroxyphenyl)-propionat) (Ciba-Geigy) oder Tocopherol (Vitamin E) in Betracht. Bevorzugt werden solche vom Typ des α-Tocopherol eingesetzt. Die Antioxidantien werden bevorzugt in Mengen von 0,15 bis 0,5 Gew.%, bezogen auf das Polyol a), eingesetzt.

Die Isocyanatkennzahl (Verhältnis der bei der Reaktion eingesetzten freien NCO-Gruppen zu den freien OH-Gruppen) der erfindungsgemäßen Polyurethangelmassen liegt je nach der Funktionalität der eingesetzten Isocyanat- und Polyolkomponenten im Bereich von 0,30 bis 0,70, bevorzugt im Bereich von 0,45 bis 0,60. Die für eine Gelbildung erforderliche Isocyanatkennzahl kann sehr einfach nach der folgenden Formel abgeschätzt werden:

$$f_{(Polyol)} \bullet (f_{(Isocyanat)} - 1) \bullet Kennzahl \approx 2$$

$$Kennzahl \approx \frac{2}{f_{(Polyol)} \bullet (f_{(Isocyanat)} - 1)}$$

f: Funktionalität der Isocyanat- oder Polyolkomponente

Je nach angestrebter Klebrigkeit oder Elastizität des Gels kann die tatsächlich zu verwendende Isocyanatkennzahl um bis zu ± 20% von dem berechneten Wert abweichen.
Die erfindungsgemäßen Polyurethangelmassen werden hergestellt nach üblichen Verfahren, wie sie beispielsweise beschrieben sind in Becker/Braun, Kunststoff- Handbuch, Bd. 7, Polyurethane, S. 121 ff, Carl-Hauser, 1983.
Weiter vorzugsweise kommen Polyurethangele zum Einsatz, wie sie in der EP 0 665 856 B1 offenbart sind. Die hydrophilen Polyurethane sind demnach erhältlich aus

1. einem Polyurethangel, welches

(A) 25-62 Gew.%, vorzugsweise 30-60 Gew.%, besonders bevorzugt 40-57 Gew.%, bezogen auf die Summe aus (A) und (B), eines kovalent vernetzten Polyurethans als hochmolekulare Matrix und
(B) 75-38 Gew.%, vorzugsweise 70-40 Gew.%, besonders bevorzugt 60-43 Gew.%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls
(C) 0 bis 100 Gew.%, bezogen auf die Summe aus (A) und (B), an Füll- und/oder Zusatzstoffen enthält,

und welches erhältlich ist durch Umsetzung einer Mischung von

a) einem oder mehreren Polyisocyanaten,
b) einer oder mehreren Polyhydroxylverbindungen mit einem mittleren Molekulargewicht zwischen 1000 und 12000, und einer mittleren OH-Zahl zwischen 20 und 112,
c) gegebenenfalls Katalysatoren oder Beschleunigern für die Reaktion zwischen Isocyanat- und Hydroxylgruppen sowie gegebenenfalls
d) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_p$) zwischen 3 und 6 beträgt und die Isocyanatkennzahl (K) der Formel

$$K = \frac{300 \pm X}{(F_I \bullet F_p) - 1} + 7$$

gehorcht, in welcher X ≤ 120, vorzugsweise X ≤ 100, besonders bevorzugt X ≤ 90 ist und die Kennzahl K bei Werten zwischen 15 und 70 liegt, wobei die angegebenen Mittelwerte von Molekulargewicht und OH-Zahl als Zahlenmittel zu verstehen sind,
2. einem Wasser absorbierenden Material und/oder
3. einem nichtwässrigen Schäumungsmittel.

Wesentlich bei der Herstellung von Polyurethanen ist jedoch, dass bei der Auswahl der gelbbildenden Komponenten die oben definierten Bedingungen eingehalten werden, da sonst anstelle von selbsthaftenden Gelen klebfreie, elastische Gele erhalten werden.
Bevorzugte Polyhydroxylverbindungen sind Polyetherpolyole, wie sie in den oben genannten Offenlegungsschriften ausführlich genannt sind.
Als Polyisocyanatkomponenten sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate bzw. hydrierte Diphenylmethandiisocyanat ("MDI")-Typen. Bevorzugte aromatischen Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen.
Die Diisocyanate können insbesondere zum Beispiel aus der Gruppe der unmodifizierten aromatischen oder

aliphatischen Diisocyanate oder aber aus durch Prepolymerisierung mit Aminen, Polyolen oder Polyetherpolyolen gebildeten modifizierten Produkten gewählt werden.

Als Vorteile der erfindungsgemäßen Polyurethane im Vergleich zu anderen Polymeren, die für die Herstellung von Verbandsmaterialien verwendet werden, lassen sich folgende Punkte nennen:

- Polyurethan stellt ein selbstklebendes System dar, wodurch auf einen Zusatz weiterer Klebstoffe, die unter Umständen Nebenwirkungen wie Mazeration, Entzündungen der dermalen Bereiche, Reduktion der Hautatmung u.a. hervorrufen, verzichtet werden kann.
- Polyurethane erweisen sich gegenüber anderen Klebematerialen, wie Polyacrylate, Kautschuk etc., als äußerst vorteilhaft, da sie kein Allergiepotential beinhalten.
- Polyurethan weist eine sehr gute Wasseraufnahmefähigkeit und Wasserdampfdurchlässigkeit auf. Hierdurch ist gewährleistet, dass bei einer Applikation über einen längeren Zeitraum keine Mazeration durch die Wasserabgabe der Haut erfolgen wird.
- Die Sauerstoffdurchlässigkeit des Polyurethans sorgt für eine gute Versorgung der abgedeckten Hautstelle mit Sauerstoff, wodurch einer Schädigung des Gewebes entgegengewirkt wird.
- Polyurethan ist allergieneutral, so dass nach der Applikation mit keiner allergischen Reaktionen des Organismus zu rechnen ist.

Die Polyurethangel-Matrix kann partiell oder vollflächig geschäumt und/oder ungeschäumt, ungefüllt oder mit zusätzlichen Füllstoffen, wie beispielsweise Superabsorbern, Titandioxid, Zinkoxid, Weichmachern, Farbstoffen etc. eingesetzt werden.
Darüber hinaus wird über die Schaumbildung ein weicheres Matrixsystem geschaffen, das für den Anwender ein positives Anfassgefühl bietet und die Herstellung eines anschmiegsameren Verbandes ermöglicht.

Vorteilhaft ist aber auch, dass das gesamte Polymermaterial ungeschäumt ist und dennoch hervorragende Anwendungseigenschaften aufweist.

Die Polyurethangele können gegebenenfalls aus der Polyurethan-Chemie an sich bekannte Zusatzstoffe enthalten, wie zum Beispiel Füllstoffe und Kurzfasern auf anorganischer oder organischer Basis, Metallpigmente, oberflächenaktive Substanzen oder flüssige Streckmittel wie Substanzen mit einem Siedepunkt von über 150°C. Als anorganische Füllstoffe seien beispielsweise Schwerspat, Kreide, Gips, Kieserit, Soda, Titandioxid, Ceroxid, Quarzsand, Kaolin, Russ und Mikrohohlkugeln genannt.

An organischen Füllstoffen können zum Beispiel Pulver auf Basis von Polystyrol, Polyvinylchlorid, Harnstoff-Formaldehyd und Polyhydrazodicarbonamid eingesetzt werden. Als Kurzfasern kommen zum Beispiel Glasfasern von 0,1 - 1 mm Länge oder Fasern organischer Herkunft, wie zum Beispiel Polyester- oder Polyamidfasern, in Frage. Metallpulver, wie zum Beispiel Eisen-, Aluminium-, oder Kupferpulver, können ebenfalls bei der Gelbildung mitverwendet werden. Um den Gelen die gewünschte Färbung zu verleihen, können die bei der Einfärbung von Polyurethanen an sich bekannten Farbstoffe oder Farbpigmente auf organischer oder anorganischer Basis verwendet werden, wie zum Beispiel Eisenoxid- oder Chromoxidpigmente, Pigmente auf Phthalocyanin- oder Monoazo-Basis. Als oberflächenaktive Substanzen seien zum Beispiel Cellulosepulver, Aktivkohle und Kieselsäurepräparate genannt.

## Patentansprüche

**1.** Verfahren zur Herstellung von Pflastern bestehend aus einer Wundauflage (6) aus selbstklebendem Material (3), einer luft- und wasserdampfdurchlässigen Barrierefolie (4) und einem Trägermaterial (9), auf dem eine Klebeschicht (8) aufgebracht ist,
**dadurch gekennzeichnet, dass**

- ein bandförmiges Laminat (1), bestehend aus Wundauflagenmaterial (3) zwischen der Barrierefolie (4) und einer Prozessfolie (2), mit der Barierefolie (4) über eine Stanzwalze (10) geführt wird,
- dabei beliebig konturierte Wundauflagen (6) ausgestanzt werden,
- die vollständig ausgestanzten Wundauflagen (6) auf eine Vakuumwalze (11) übertragen werden,
- die Wundauflagen (6) von der Vakuumwalze (11) mit der Barrierefolie (4) auf ein Trägermaterial (9), das mit einer Klebeschicht (8) beschichtet ist, überführt werden,

    - wobei die Fördergeschwindigkeit des Trägermaterials (9) gegenüber der Umlaufgeschwindigkeit der vanuumwatze (11) höher ist,

- mittels einer Vakuumwalze (12) die Prozessfolie (2) vom Wundauflagenmaterial (3) entfernt wird und

- abschließend die Pflaster entsprechend vereinzelt und gegebenenfalls eingedeckt und versiegelt werden.

2. Verfahren nach Anspruch 1, **dadurch gekennzeichnet, dass** das Entfernen der Prozessfolie (2) vom Wundauflagenmaterial (3) durch Krümmungsführung und einer Umlenkrolle (15) unterstützt wird.

3. Verfahren nach Anspruch 1 oder 2, **dadurch gekennzeichnet, dass** alle Verfahrensschritte durch Markierungen auf den verwendeten Folienmaterialien, Lichtschranken, Schrittmotoren und/oder Steuereinheiten aufeinander abgestimmt werden.

4. Verfahren nach einem der vorstehenden Ansprüche, **dadurch gekennzeichnet, dass** die Ausstanzwalze (10) auswechselbar ist.

5. Verfahren nach einem der vorstehenden Ansprüche, **dadurch gekennzeichnet, dass** als Wundauflagenmaterial ein elastisches Material verwendet wird.

6. Verfahren nach Anspruch 5, **dadurch gekennzeichnet, dass** als Wundauflagenmaterial Polyurethan verwendet wird.

7. Verfahren nach einem der vorstehenden Ansprüche, **dadurch gekennzeichnet, dass** als Barrierefolie (4) und/oder Trägermaterial (9) Polyurethane verwendet werden.

## Claims

1. Process for the manufacture of plasters composed of a wound contact pad (6) made of self-adhesive material (3), an air- and water-vapour-permeable barrier film (4) and a backing material (9), applied atop which there is an adhesive layer (8),
**characterized in that**

    - a tapelike laminate (1), composed of wound contact material (3) between the barrier film (4) and an in-process film (2), is passed with the barrier film (4) over a diecutting roll (10),
    - in the course of which wound contact pads (6) of any desired contour are diecut,
    - the fully diecut wound contact pads (6) are transferred to a vacuum roll (11),
    - the wound contact pads (6) are transferred from the vacuum roll (11) with the barrier film (4) to a backing material (9) which has a coating of an adhesive layer (8),

        - the conveying speed of the backing material (9) being higher than the circulating speed of the vacuum roll (11),

    - the in-process film (2) is removed from the wound contact material (3) by means of a vacuum roll (12), and
    - lastly the plasters are appropriately separated and, if desired, are covered and sealed.

2. Process according to Claim 1, **characterized in that** the removal of the in-process film (2) from the wound contact material (3) is assisted by curved passage and a deflection roller (15).

3. Process according to Claim 1 or 2, **characterized in that** all steps of the process are harmonized with one another by means of markings on the film materials used, light barriers, stepping motors and/or control units.

4. Process according to any one of the preceding claims, **characterized in that** the diecutting roll (10) is exchangeable.

5. Process according to any one of the preceding claims, **characterized in that** an elastic material is used as wound contact material.

6. Process according to Claim 5, **characterized in that** polyurethane is used as wound contact material.

7. Process according to any one of the preceding claims, **characterized in that** polyurethanes are used as barrier film (4) and/or backing material (9).

## Revendications

1. Procédé pour la préparation de sparadraps constitués par un pansement (6) en matériau auto-adhésif (3), une feuille (4) formant une barrière perméable à l'air et à la vapeur d'eau et un matériau support (9), sur lequel est appliquée une couche adhésive (8), **caractérisé en ce**

   - **qu'**on guide un laminé (1) en forme de bande, constitué par le matériau pour pansement (3) entre la feuille (4) formant la barrière et une feuille (2) de procédé, avec la feuille (4) formant la barrière sur un cylindre de découpe (10),
   - en découpant des pansements (6) présentant un contour quelconque,
   - **qu'**on applique les pansements (6) complètement découpés sur un cylindre sous vide (11),
   - **qu'**on transfère les pansements (6) du cylindre sous vide (11) avec la feuille (4) formant une barrière sur un matériau support (9) qui est revêtu d'une couche adhésive (8)

     - la vitesse d'avancement du matériau support (9) étant supérieure à la vitesse de rotation du cylindre sous vide (11),

   - **qu'**un cylindre sous vide (12) élimine la feuille (2) de procédé du matériau formant le pansement (3) et
   - **que** les sparadraps sont enfin séparés et le cas échéant recouverts et scellés.

2. Procédé selon la revendication 1, **caractérisé en ce que** l'élimination de la feuille de procédé (2) du matériau (3) formant le pansement est soutenue par un guidage courbé et une poulie de déviation (15).

3. Procédé selon la revendication 1 ou 2, **caractérisé en ce que** toutes les étapes de procédé sont adaptées les unes aux autres par des marquages sur les matériaux pour feuille, les détecteurs photo-électriques, les moteurs pas à pas et/ou les unités de commande utilisés.

4. Procédé selon l'une quelconque des revendications précédentes, **caractérisé en ce que** le cylindre de découpe (10) peut être remplacé.

5. Procédé selon l'une quelconque des revendications précédentes, **caractérisé en ce qu'**on utilise comme matériau formant le pansement un matériau élastique.

6. Procédé selon la revendication 5, **caractérisé en ce qu'**on utilise comme matériau formant le pansement du polyuréthane.

7. Procédé selon l'une quelconque des revendications précédentes, **caractérisé en ce qu'**on utilise comme feuille (4) formant une barrière et/ou comme matériau support (9) des polyuréthanes.

Zeichnungen